# EUROPÄISCHE PATENTSCHRIFT

(11) **EP 1 513 570 B1**
(45) Veröffentlichungstag und Bekanntmachung des Hinweises auf die Patenterteilung: **15.11.2006**
(21) Anmeldenummer: 03740107.2
(22) Anmeldetag: 19.06.2003
(51) Int. Cl.: A61M 5/14, A61M 39/02

(54) **INFUSIONSSAMMLER**
INFUSION COLLECTOR
COLLECTEUR DE PERFUSION

(30) Priorität: 20.06.2002 DE 20209573 U
(43) Veröffentlichungstag der Anmeldung: 16.03.2005
(73) Patentinhaber: Georg-August-Universität Göttingen, 37073 Göttingen (DE)
(72) Erfinder: RADKE, Oliver, C., 37085 Göttingen (DE)
(74) Vertreter: Stornebel, Kai
(86) Internationale Anmeldenummer: PCT/DE2003/002092
(87) Internationale Veröffentlichungsnummer: WO 2004/000393

(56) Entgegenhaltungen:
- EP-A- 1 043 038
- WO-A-01/36026
- WO-A-99/62583
- US-A- 4 439 182
- US-A- 4 447 230
- US-A- 5 405 323
- US-A- 5 807 312
- US-A- 6 102 889

## Beschreibung

Die Erfindung betrifft einen Infusionssammler mit den Merkmalen des Oberbegriffs des Anspruchs 1 zum Zusammenführen und Mischen mehrerer Infusions- und/oder Injektionsflüssigkeiten für eine totale intravenöse Anästhesie (TIVA), mit einem einzigen venösen Zugang zum Patienten und mehreren Anschlüssen für die Infusions- und/oder Injektionsflüssigkeiten.

Die EP 0 992 257 A1 offenbart einen Infusionssammler, der mit zahlreichen Nachteilen behaftet ist. So fehlt ein direkter Flussweg der Basisinfusion; das dünne Lumen sowie eingebrachte Filter stören den Fluss und stellen einen hohen Widerstand dar. Vorgesehen sind Rückschlagventile, die so angeordnet sind, dass bei geringem Fluss oder Flüssigkeitsstillstand Leckagen und Kontaminationen durch Diffusion auftreten können. Im Hauptstrom ist nur ein Ventil vorgesehen, so dass ein Schutz vor Kontamination durch Patientenblut nicht gegeben ist. Die Ausführungsform ist überdies sehr klobig und daher nur schwer handhabbar.

In dem US-Patent 4,439,182 ist ein Infusionssammler mit einer Infusionsleitung beschrieben, bei dem in die Infusions- und/oder Injektionsflüssigkeit in einen an einem ersten Ende der Infusionsleitung angeordneten Behälter hineingetropft wird. In der Infusionsleitung sind Spritzenanschlüsse zur Injektion von Medikamenten vorgesehen.

Die EP 1 043 038 A2 offenbart ein aus Einzelteilen bedarfsweise zusammenbaubares Kombinationssystem, bei dem Infusions- und Perfusorleitungen an ihrem Ende jeweils über eine Rückflussbarriere an zugeordnete Mehrwegehähne angeschlossen werden. Diese Mehrwegehähne werden so zusammengesteckt, dass die Perfusorleitungen in den Strömungsweg der Infusorleitung einmünden.

Der Erfindung liegt die Aufgabe zugrunde, die Handhabung eines Infusionssammlers zu vereinfachen, bei dem zwei Medikamente einer laufenden Infusion zugeführt werden können.

Zur Lösung dieser Aufgabe ist ein Infusionssammler der eingangs erwähnten Art erfindungsgemäß gekennzeichnet, durch die kennzeichnenden Merkmale des Anspruchs 1.

Das erfindungsgemäße TIVA-Set ermöglicht ohne zusätzliche Teile ein Zusammenführen von Infusion und zwei Medikamenten aus Spritzenpumpen in einen venösen Zugang. Die an dem patientenfemen Ende der beiden Perfusorleitungen vorgesehenen Ventile und optional das patientennahe zweite Ventil verhindern einen Rückfluss von Patientenblut und beugt dadurch einer Kontamination der Medikamente in den Spritzenpumpen vor. Dabei dient die kurze Verlängerung zwischen dem T-Stück und dem venösen Zugang am Patienten als Entlastung, um Hebelkräfte am venösen Zugang zu vermeiden. Die Ventile an den Perfusorleitungen ermöglichen andererseits ein umkompliziertes Wechseln bzw. Nachfüllen der Perfusorspritzen, die ohne weiteres diskonnektiert und wieder aufgesetzt werden können.

Das TIVA-Set ermöglicht somit die Durchführung einer TIVA über einen einzigen venösen Zugang. Die Perfusorleitungen erlauben Flüsse von über 800 ml/h, so dass auch die Bolusgabe aus den Perfusoren zur Narkoseinduktion möglich ist. Nach Beendigung der Narkose wird die kurze Verlängerung mit dem Ventil am venösen Zugang belassen und die Infusionsleitung am Ventil rekonnektiert. Das TIVA-Set wird verworfen, da eine Kontamination nicht mit völliger Sicherheit ausgeschlossen werden kann. Die Medikamentenspritzen hingegen sind durch die verhältnismäßig große Länge der Perfusorleitungen sowie durch deren Ventile geschützt und können daher weiter benutzt werden.

Die Materialkosten für ein erfindungsgemäßes TIVA-Set sind erheblich geringer als die für einen Selbstbau benötigten Komponenten. Bei einer Narkoseinduktion über die Bolus-Funktion der Perfusoren entfallen zusätzlich die Kosten für Einmalspritzen und Nullstopfen. Die Medikamentenreste aus den Perfusoren können weiter benutzt werden; bei Induktion aus den Perfusoren müssen keine Reste der Induktionsdosis verworfen werden. Da das erfindungsgemäße TIVA-Set fertig vorbereitet ist, werden Vorbereitungs- und Überleitungszeiten eingespart. Im Übrigen erleichtert das erfindungsgemäße TIVA-Set eine Standardisierung der Abläufe und erhöht auch dadurch die Sicherheit für den Patienten.

Bevorzugt werden bei der Erfindung hämostatische Ventile eingesetzt, die im Gegensatz zu herkömmlichen Rückschlagventilen (hämodynamische Ventile) bereits bei Strömungsstillstand schließen und nicht einen Gegendruck zum Schließen benötigen. Dadurch kann eine noch höhere Sicherheit gegen eine retrograde Diffusion von Keimen o.ä. in das System erzielt werden.

Für die erleichterte Handhabung des Infusionssammlers ist es zweckmäßig, wenn die kurze Verlängerung mit dem ihr zugeordneten Anschluss des T-Stückes über eine Verschraubung lösbar verbunden ist, und wenn das zweite, patientennahe Ventil von dem freien Ende der kurzen Verlängerung abschraubbar ist.

Zur Ermöglichung einer hohen Flussrate ist es vorteilhaft, wenn der Strömungsquerschnitt des den ersten Anschluss des T-Stückes mit seinem zweiten Anschluss verbindenden Strömungsweges etwa dem der Infusionsleitung entspricht.

Ferner ist es vorteilhaft, wenn die Perfusorleitungen gegenüber der Infusionsleitung - und somit gegenüber dem Strömungsweg in dem T-Stück - sehr kleinlumig ausgebildet sind.

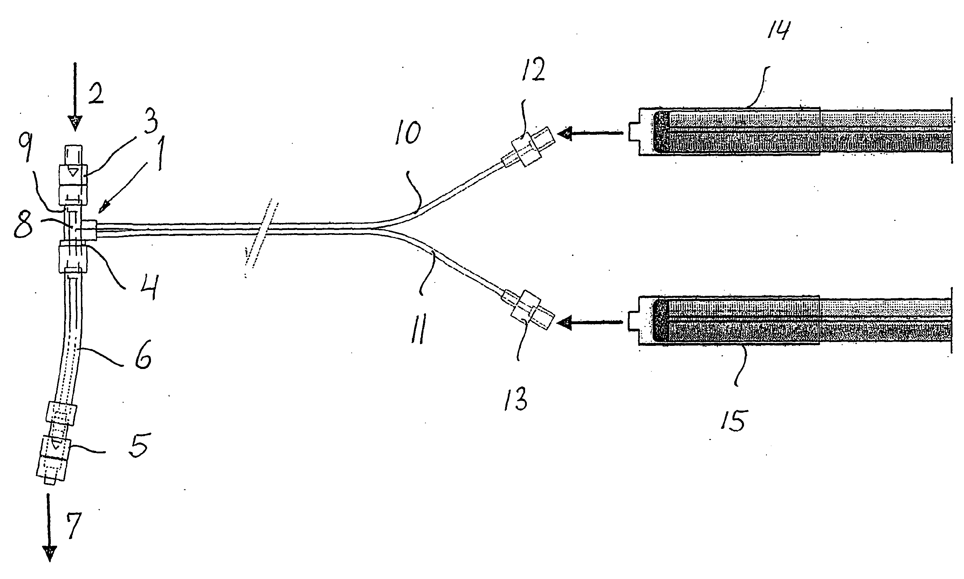

In der Zeichnung ist eine als Beispiel dienende Ausführungsform der Erfindung dargestellt.

Die Zeichnung zeigt einen Infusionssammler mit einem T-Stück 1, das zum Anschluss einer Infusionsleitung 2 ein erstes Ventil 3 und diesem in Strömungsrichtung gegenüberliegend einen Anschluss 4 für eine kurze, an ihrem freien Ende mit einem zweiten Ventil 5 bestückte Verlängerung 6 zum venösen Zugang am Patienten (Pfeil 7) aufweist.

In das T-Stück 1 münden in den Strömungsweg 8 zwischen dem ersten Anschluss 9 und dem zweiten Anschluss 4 zwei Perfusorleitungen 10, 11 ein, die an ihrem freien Ende jeweils mit einem dritten Ventil 12 bzw. vierten Ventil 13 bestückt sind. An das dritte Ventil 12 ist eine Perfusorspritze 14 für ein Medikament a und an das vierte Ventil 13 eine zweite Perfusorspritze 15 für ein Medikament b ansetzbar.

Die kurze Verlängerung 6, die eine Länge von etwa 15 cm aufweisen kann, ist mit dem ihr zugeordneten Anschluss 4 des T-Stücks 1 über eine Verschraubung 16 lösbar verbunden.

Das zweite, patientennahe Ventil 5 ist von dem freien Ende der kurzen Verlängerung 6 abschraubbar.

Der Strömungsquerschnitt des den ersten Anschluss 9 des T-Stücks 1 mit seinem zweiten Anschluss 4 verbindenden Strömungsweges 8 entspricht etwa dem der Infusionsleitung 2, dergegenüber die Perfusorleitungen 10, 11 sehr kleinlumig ausgebildet sind. Die Länge jeder Perfusorleitung 10, 11 beträgt etwa 2 m.

## Patentansprüche

1. Infusionssammler zum Zusammenführen und Mischen mehrerer Infusions- und/oder Injektionsflüssigkeiten für eine totale intravenöse Anästhesie (TIVA) in Form eines vorkonfigurierten Produkts (nachfolgend "TIVA-Set") mit einem ersten Anschluss (9) für einen Infusionsleiter (2), eine flexible kurze Verlängerungsleitung (6), die an ihrem freien Ende mit einem zweiten Ventil (5) bestückt ist und einen Anschluss für einen venösen Zugang (7) aufweist und mit zwei Perfusorleitungen (10, 11), die in den Strömungsweg (8) zwischen den Anschlüssen einmünden, **dadurch gekennzeichnet, dass** der erste Anschluss (9) an einem T-Stück (1) ausgebildet und mit einem ersten Ventil (3) bestückt ist,
das T-Stück (1) in Strömungsrichtung dem ersten Anschluss (9) gegenüberliegend einen zweiten Anschluss (4) aufweist, an den die Verlängerungsleitung (6) angeschlossen ist, und das T-Stück (1) in den Strömungsweg (8) zwischen seinem ersten und zweiten Anschluss (9, 4) die beiden Perfusorleitungen (10, 11) einmünden, die an ihrem freien Ende jeweils mit einem dritten bzw, vierten Ventil (12, 13) zum lösbaren Ansetzen je einer Perfursorspritze (14, 15) bestückt sind.

2. Infusionssammler nach Anspruch 1, **dadurch gekennzeichnet, dass** die kurze Verlängerung (6) mit dem ihr zugeordneten Anschluss (4) des T-Stücks (1) über eine Verschraubung (16) lösbar verbunden ist.

3. Infusionssammler nach Anspruch 1 oder 2, **dadurch gekennzeichnet, dass** das zweite, patientennahe Ventil (5) von dem freien Ende der kurzen Verlängerung (6) abschraubbar ist.

4. Infusionssammler nach einem der Ansprüche 1 bis 3, **dadurch gekennzeichnet, dass** der Strömungsquerschnitt des den ersten Anschluss (9) des T-Stücks (1) mit seinem zweiten Anschluss (4) verbindenden Strömungswegs (8) deutlich größer als der der Perfusorleitungen (10, 11) ist.

5. Infusionssammler nach einem der Ansprüche 1 bis 4, **dadurch gekennzeichnet, dass** die Ventile (3, 5, 12, 13) als hämostatische Ventile ausgebildet sind.

6. Infusionssammler nach einem der Ansprüche 1 bis 5, **dadurch gekennzeichnet, dass** die Länge der Verlängerungsleitung (6) wesentlich kleiner ist als die Länge der Perfusorleitungen (10, 11).

7. Infusionssammler nach Anspruch 6, **dadurch gekennzeichnet, dass** die Länge der kurzen Verlängerung (6) etwa 15 cm beträgt.

8. Infusionssammler nach Anspruch 6 oder 7, **dadurch gekennzeichnet, dass** die Länge jeder Perfusorleitung (10, 11) etwa 2 m beträgt.

## Claims

1. Infusion collector for bringing together and mixing several infusion and/or injection liquids for total intravenous anaesthesia (TIVA), in the form of a pre-configured product (hereinafter TIVA set), with a first connection (9) for an infusion line (2), a flexible short extension line (6) which is equipped at its free end with a second valve (5) and which has a connection for a venous access (7), and with two perfusion lines (10, 11) which open into the flow path (8) between the connections, **characterized in that** the first connection (9) is formed on a T-piece (1) and is equipped with a first valve (3), the T-piece (1) has a second connection (4) which lies opposite the first connection (9) in the direction of flow and to which the extension line (6) is connected, and the two perfusion lines (10, 11) open into the T-piece (1), into the flow path (8) between its first and second connections (9, 4), said two perfusion lines (10, 11) being equipped at each of their free ends with a third valve (12) and fourth valve (13) respectively, for releasable attachment of a respective perfusion syringe (14, 15).

2. Infusion collector according to Claim 1, **characterized in that** the short extension (6) is releasably connected to its associated connection (4) on the T-piece (1) via a screw union (16).

3. Infusion collector according to Claim 1 or 2, **characterized in that** the second valve (5) near the patient can be unscrewed from the free end of the short extension (6).

4. Infusion collector according to one of Claims 1 to 3, **characterized in that** the cross section of flow of the flow path (8) joining the first connection (9) of the T-piece (1) to its second connection (4) is much greater than that of the perfusion lines (10, 11).

5. Infusion collector according to one of Claims 1 to 4, **characterized in that** the valves (3, 5, 12, 13) are designed as haemostatic valves.

6. Infusion collector according to one of Claims 1 to 5, **characterized in that** the length of the extension line (6) is considerably smaller than the length of the perfusion lines (10, 11).

7. Infusion collector according to Claim 6, **characterized in that** the length of the short extension (6) is approximately 15 cm.

8. Infusion collector according to Claim 6 or 7, **characterized in that** the length of each perfusion line (10, 11) is approximately 2 m.

## Revendications

1. Collecteur d'infusion pour rassembler et mélanger plusieurs infusions et/ou liquides d'injection pour une anesthésie intraveineuse totale (AIVT) sous la forme d'un produit préconfiguré (ci-après set-AIVT) comportant un premier raccord (9) pour une tubulure d'infusion (2), une tubulure flexible courte de prolongement (6) qui porte à son extrémité libre une deuxième soupape (5) et présente un raccord pour un accès veineux (7) et deux tubulures de perfusion (10, 11) qui débouchent dans le passage de circulation (8) entre les raccords, **caractérisé en ce que** le premier raccord (9) est réalisé sur une pièce en T (1) et porte une première soupape (3), la pièce en T (1) présente à l'opposé du premier raccord (9) auquel est raccordé la tubulure de prolongement (6) et les deux tubulures de perfusion (10, 11) débouchent toutes deux dans la pièce en T (1) dans le passage de circulation (8) entre son premier et deuxième raccord (9,4), celles-ci comportant à chacune de leurs extrémités libres une troisième ou une quatrième soupape (12, 13) pour la connexion amovible à chacune d'elles d'une seringue de perfusion (14, 15).

2. Collecteur d'infusion selon la revendication 1, **caractérisé en ce que** le prolongement court (6) est relié de façon amovible au raccord (4) correspondant de la pièce en T (1) au moyen d'un vissage (16).

3. Collecteur d'infusion selon la revendication 1 ou 2, **caractérisé en ce que** la deuxième soupape (5) proche du patient peut être dévissée de l'extrémité libre du prolongement court (6).

4. Collecteur d'infusion selon l'une des revendications 1 à 3, **caractérisé en ce que** la section d'écoulement du passage de circulation (8) reliant le premier raccord (9) de la pièce en T (1) à son second raccord (4) est significativement plus grande que celle des tubulures de perfusion (10, 11).

5. Collecteur d'infusion selon l'une des revendications 1 à 4, **caractérisé en ce que** les soupapes (3, 5, 12, 13) sont conformées en soupapes hémostatiques.

6. Collecteur d'infusion selon l'une des revendications 1 à 5, **caractérisé en ce que** la longueur de la tubulure de prolongement (6) est nettement plus courte que la longueur des tubulures de perfusion (10, 11).

7. Collecteur d'infusion selon la revendication 6, **caractérisé en ce que** la longueur du prolongement court (6) est de l'ordre de 15 cm.

8. Collecteur d'infusion selon la revendication 6 ou 7, **caractérisé en ce que** la longueur de chaque tubulure de perfusion (10, 11) est de l'ordre de 2m.
